# FASCICULE DE BREVET EUROPEEN

(11) **EP 1 795 138 B1**
(45) Date de publication et mention de la délivrance du brevet: **10.03.2010**
(21) Numéro de dépôt: 06301140.7
(22) Date de dépôt: 09.11.2006
(51) Int. Cl.: A61B 17/72

(54) **Clou centromedullaire d'osteosynthese**
Knochenmarknagel für die Osteosynthese
Intramedullary nail for osteosynthesis

(30) Priorité: 09.11.2005 FR 0553393
(43) Date de publication de la demande: 13.06.2007
(73) Titulaire: Société de Génie Médical - SGM, 42000 Saint Etienne (FR); Collin, Philippe, 35590 Clayes (FR); Julien, Yann, 21240 Talant (FR); Beguin, Laurent, 69540 Irigny (FR); Desmoineaux, Pierre, 78150 Le Chesnay (FR)
(72) Inventeur: Collin, Philippe, 35590, CLAYES (FR); Julien, Yann, 21240, TALANT (FR); Beguin, Laurent, 69540, IRIGNY (FR); Desmoineaux, Pierre, 78150, LE CHESNAY (FR); Colombier, Michel, 42270 Saint Priest en Jarez (FR)
(74) Mandataire: Thivillier, Patrick

(56) Documents cités:
- EP-A- 1 391 187
- WO-A-02/080790
- DE-A1- 10 204 904
- US-A1- 2004 122 428

## Description

L'invention concerne le domaine technique des clous centromédullaires d'ostéosynthèse.

Plus particulièrement, l'invention concerne un clou pour le traitement des fractures de la tête humérale sans tubérositaires 3 ou 4 fragments.

D'une manière parfaitement connue pour un homme du métier, un clou d'ostéosynthèse est destiné à être engagé dans le canal médullaire de l'os considéré en y étant fixé, notamment en partie proximale, au moyen de vis engagées dans des trous débouchant formés dans ladite partie proximale. Ces trous peuvent être lisses ou taraudés. Les vis engagées au travers des trous sont généralement vissées dans l'os spongieux.
Le clou peut également être utilisé pour des fractures diaphysaires ou des fractures associées diaphysaires et proximales. Dans ce cas, lesdites vis peuvent être engagées dans des trous formés au niveau de la partie distale du clou et vissées dans la corticale.
Le clou, réalisé sous forme d'une tige cylindrique creuse, généralement en acier, peut être rectiligne et présenter, sensiblement au niveau de sa partie distale des pans coupés pour faciliter son introduction dans l'os en approche percutanée.

On observe également qu'en partie proximale les trous recevant les vis, peuvent être alignés ou décalés angulairement afin d'orienter, d'une manière correspondante, les vis dans l'os spongieux en fonction du type de fracture à traiter et surtout renforcer l'effet anti-basculement de la tête humérale pendant sa consolidation.

A partir de cette conception de base, d'un clou d'ostéosynthèse, de nombreuses améliorations techniques ont été apportées notamment pour réduire les micromouvements susceptibles de résulter de différentes sollicitations auxquelles est soumis l'os. Par exemple, il ressort de l'enseignement du brevet FR 2.784.283 qu'au moins l'un des trous recevant une vis en partie proximale est lisse et présente transversalement une tige interne faisant saillie à l'intérieur dudit trou, de manière à faire office d'appui au filet de la vis. Ces dispositions permettent de bloquer le clou par rapport à la vis dans la direction axiale de cette dernière et entre le décentrage de l'implant.

On peut citer également les documents WO 02/080790 qui concerne un clou centromédullaire dont la partie proximale reçoit une bague externe fixée par une patte à l'extérieur de la tête fémorale, US 2004/0122428 qui concerne un clou coopérant avec un moyen externe de centrage et DE 102 04 904 qui concerne un clou dont le calage s'effectue en partie distale.

A partir de cet état de la technique, l'invention se propose de résoudre d'une autre façon, d'une part, le problème de calage et de centrage du clou dans le canal médullaire de l'os, notamment en partie métaphysaire et, d'autre part, le problème du basculement de la tête humérale en varisation sous l'effet des tractions des tubérosités à consolider

Pour résoudre ces problèmes, il a été conçu un clou centromédullaire conforme aux caractéristiques de la revendication 1.

Dans une forme de réalisation de l'invention, le ou les moyens rapporté(s) est/sont constitué(s) par une ou des bagues externe(s) emmanchée(s) sur la périphérie dudit clou pour être engagée(s) dans le canal médullaire, après impaction.

Selon une autre forme de réalisation, le ou les moyens rapporté(s) est/sont constitué(s) par au moins une bague et au moins une agrafe présentant des agencements pour l'engagement d'une vis de fixation commune en partie proximale. La vis de fixation est bloquée dans le sens transversal par rapport au clou par un système vis écrou dont la particularité est que l'écrou est à un pas. L'immobilisation clou - vis contrairement à l'agencement développé dans le brevet FR 2784283 est obtenue grâce au filet de l'écrou réalisé à l'intérieur du trou de passage de la vis pour immobiliser la vis sur le clou, le trou fileté ne présentant qu'un pas de filet.

Un autre problème que se propose de résoudre l'invention est de faciliter la mise en place du clou dans le canal médullaire en diminuant l'ouverture d'introduction et en ayant une approche percutanée. Dans ce but, pour résoudre un tel problème, l'extrémité distale du clou est conformée en pointe du type carré.

L'invention est exposée ci-après plus en détail à l'aide des figures des dessins annexés dans lesquels :
- la figure 1 est une vue en perspective du clou avant mise en place de l'agrafe en matériau souple et engagement de la vis proximale ;
- la figure 2 est une vue correspondant à la figure 1 après mise en place de l'agrafe et engagement de la vis proximale ;
- la figure 3 est une vue en perspective du clou avant mise en place de l'agrafe selon une autre forme de réalisation ;
- la figure 4 est une vue en perspective correspondant à la figure 3 après mise en place de l'agrafe souple et engagement de la vis proximale ;
- la figure 5 est une vue en perspective d'une autre forme de réalisation du clou, avant mise en place de bagues de centrage montées en combinaison avec une agrafe rigide maintenues ensemble par une vis à os spongieux ;
- la figure 6 est une vue en perspective correspondant à la figure 5 après mise en place des bagues de centrage et de l'agrafe maintenue par les vis à os spongieux.
- la figure 7 est une vue transversale de la définition d'un des trous du clou ;
- la figure 8 est une vue transversale du trou du clou avec sa vis de fixation précisant l'intérêt de l'écrou à un pas.

Le clou centromédullaire est réalisé, d'une manière parfaitement connue pour un homme du métier, à partir d'une tige cylindrique (1) de section creuse et réalisée en métal notamment. L'extrémité proximale (1a) du clou (1) présente des agencements pour coopérer avec, par exemple, un viseur.
Selon l'invention, l'extrémité distale (1b) est conformée en pointe, du type carré, pour permettre une approche percutanée afin, notamment, d'éviter de réaliser, au niveau de la partie proximale de l'os constitué par exemple par la tête humérale, une ouverture ou un alésage.

Selon une autre caractéristique, le clou (1) coopère, notamment au niveau de la partie proximale, avec des moyens externes rapportés aptes à assurer son centrage et son calage latéral dans le canal médullaire de l'os considéré, l'humérus par exemple. Ces moyens peuvent être constitués par au moins une bague ou bouchon (2) conformé pour être emmanché sur la périphérie externe du clou (1). Le diamètre externe de la bague (2) est conformé pour être coincé dans le canal huméral par exemple, pour assurer, d'une manière concomitante, l'auto-centrage et le calage du clou.

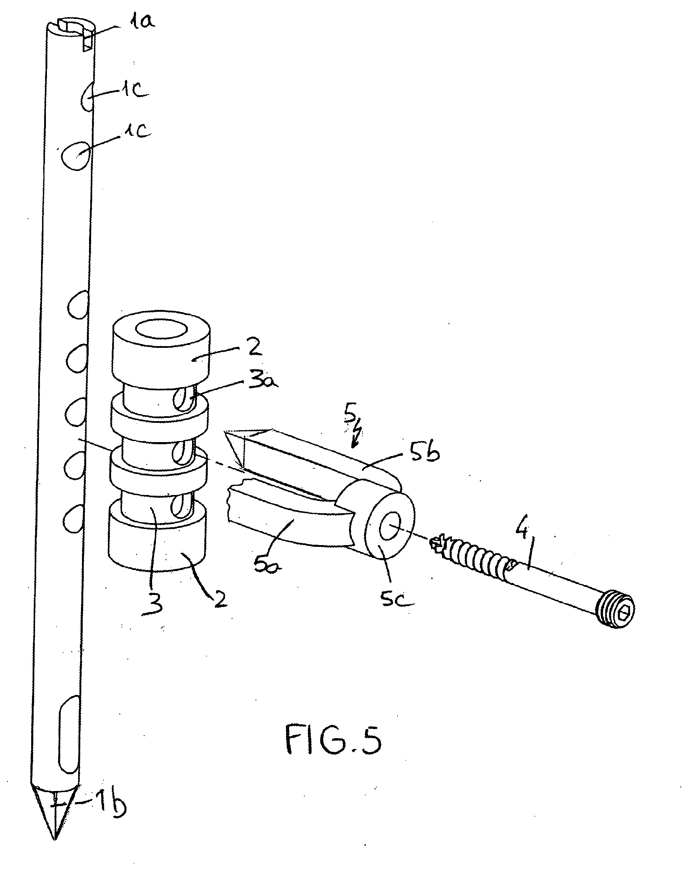

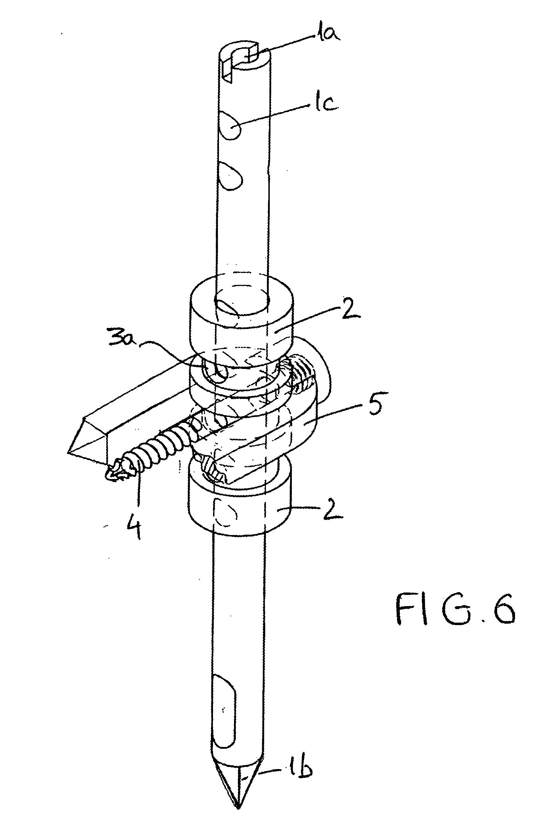

Comme le montrent les figures 5 et 6, la partie proximale du clou (1) peut recevoir plusieurs bagues (2) formées à partir d'un fourreau commun (3) apte à être emmanché sur la périphérie dudit clou. Le fourreau (3) présente des trous (3a) pour le passage des vis à os spongieux (4) aptes à être engagées au travers de trous (1c) que présente, d'une manière parfaitement connue, la partie proximale du clou (1). Les trous (1c) peuvent être alignés ou décalés angulairement, comme indiqué précédemment. De même, les vis à spongieux (4) sont de tout type connu et approprié. La ou les bague(s) (2) peu(ven)t être en matériau résorbable.

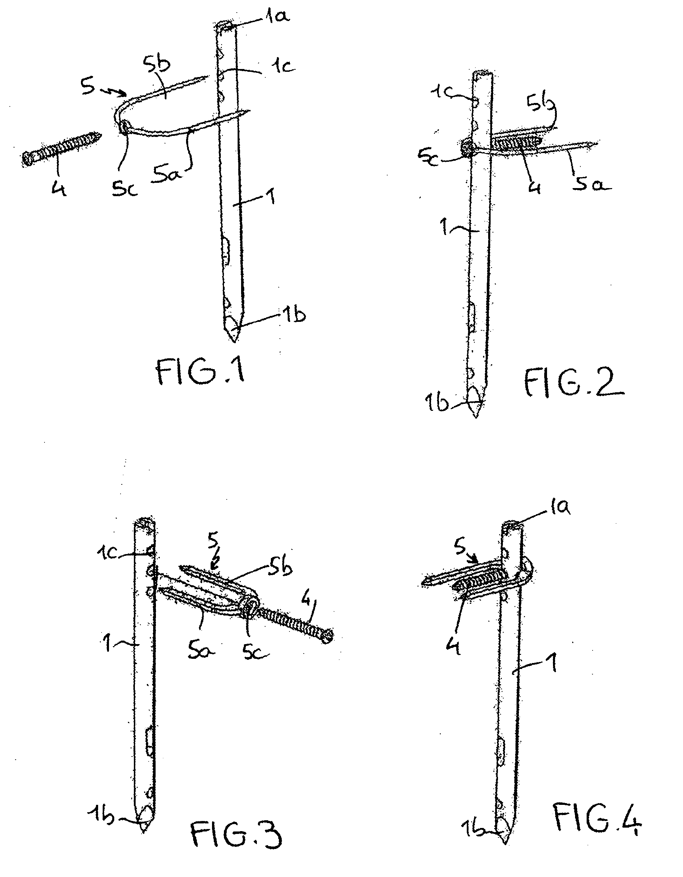

Selon une autre forme de réalisation (figures 1 à 4), pour assurer le calage et le centrage du clou dans le canal médullaire de l'os, on peut utiliser une agrafe (5). Cette agrafe (5) présente au moins deux branches (5a) et (5b) réunies par une partie commune (5c) présentant des agencements pour l'engagement notamment de la vis à spongieux (4). Dans une variante, la tête de vis peut être filetée afin d'obtenir un ensemble rigide vis - clou - agrafe.
Aux figures 1 et 2, l'agrafe (1) est réalisée dans un matériau souple notamment à mémoire de forme.
Aux figures 3 et 4, l'agrafe est réalisée dans un matériau rigide. On observe également que l'agrafe rigide peut faire office d'appui grâce à la forme aplatie de la branche, quand la tête humérale a basculé.

Eventuellement, on peut monter en combinaison avec le clou (1), les bagues (2) et l'agrafe (5). Dans ce cas, comme le montrent les figures 5 et 6, les branches (5a) et (5b) de l'agrafe sont engagées autour du fourreau (3), dans l'intervalle laissé libre par deux bagues. Dans ce cas, le fourreau (3) est percé de part en part pour permettre l'engagement de la vis (4).

A noter que la tête de vis (4) peut être sphérique ou filetée à pas différentiel, notamment pour lier l'ensemble agrafe - vis.

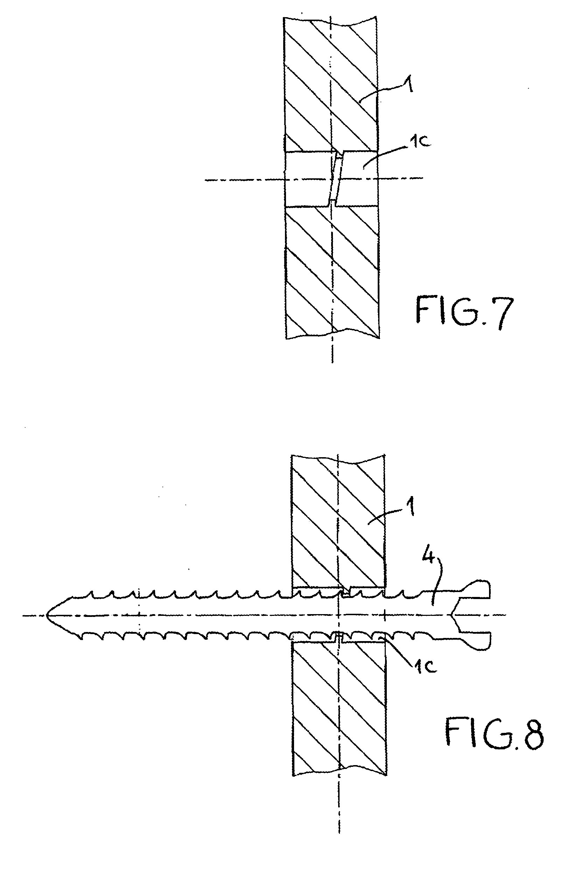

La définition du trou de passage (1c) de la vis (4) est telle qu'elle présente un filet de un pas qui fait office d'ensemble écrou/vis pour immobiliser transversalement le clou par rapport à la vis. L'intérêt de l'écrou à un pas permet de recevoir l'âme de la vis sans grippage ou blocage intempestif (figures 7 et 8).

Le clou (1) peut être entièrement rectiligne ou présenter une courbure notamment au niveau proximal.

Les avantages ressortent bien de la description.

## Revendications

1. Clou centromédullaire d'ostéosynthèse destiné à être engagé dans le canal médullaire d'un os en y étant fixé en partie proximale, et éventuellement distale, par des vis (4), ledit clou présentant, au niveau de sa partie proximale (1a), un ou des moyen(s) apte(s), d'une part, à assurer le centrage et le calage latéral dudit clou (1) dans ledit canal et, d'autre part, à assurer la rigidité de la tête humérale par rapport au corps de l'os,
**caractérisé en ce que** le ou les moyens rapporté(s) est/sont constitué(s) par une agrafe ou plusieurs agrafes (5).

2. Clou selon la revendication 1, **caractérisé en ce que** l'agrafe (5) présente au moins deux branches (5a) et (5b) réunies par une partie commune (5c) présentant des agencements pour l'engagement ou la fixation des vis de fixation (4) en partie proximale.

3. Clou selon l'une quelconque des revendications 1 et 2, **caractérisé en ce que** l'agrafe (5) est réalisée dans un matériau souple à mémoire de forme.

4. Clou selon l'une quelconque des revendications 1 et 2, **caractérisé en ce que** l'agrafe (5) est réalisée dans un matériau rigide.

5. Clou selon la revendication 1, **caractérisé en ce que** le ou les moyens rapporté(s) est/sont constitué(s) par au moins une bague (2) et au moins une agrafe (5) présentant des agencements pour l'engagement d'une vis de fixation commune (4) commune en partie proximale.

6. Clou selon la revendication 5, **caractérisé en ce que**, dans le cas de plusieurs bagues (2), ces dernières sont formées à partir d'un fourreau (3) apte à être emmanché sur la périphérie dudit clou (1), les branches (5a) et (5b) de l'agrafe étant engagées autour du fourreau (3), dans l'intervalle laissé libre par deux bagues, ledit fourreau (3) présente des trous (3a) pour le passage des vis (4) en correspondance avec des trous (1c) du clou.

7. Clou selon la revendication 6, **caractérisé en ce que** le fourreau (3) est réalisé dans une matière résorbable.

8. Clou selon l'une quelconque des revendications 1 à 7, **caractérisé en ce que** l'extrémité distale (1b) du clou est conformée en pointe du type carré pour permettre une approche percutanée.

9. Clou selon la revendication 1, **caractérisé en ce que** le ou les trous de passage (1c) recevant la ou les vis (4) présent(ent) un filet de un pas qui fait office d'ensemble écrou-vis.

## Claims

1. Centro-medullary nail for osteosynthesis intended to be engaged in the medullary canal of a bone by being secured thereto in the proximal, and possibly distal, part, by screws (4), said nail having, in its proximal part (1a), one or more means capable, on the one hand, of centring and laterally wedging said nail (1) in said canal and, on the other hand, of rigidifying the humeral head relative to the body of the bone,
**characterized in that** the added means is/are constituted by a clip or a plurality of clips (5).

2. Nail as claimed in claim 1, **characterized in that** the clip (5) has at least two branches (5a) and (5b) joined by a common part (5c) having arrangements for the engagement or attachment of the attachment screws (4) in the proximal part.

3. Nail as claimed in any one of claims 1 and 2, **characterized in that** the clip (5) is made out of a shape-memory flexible material.

4. Nail as claimed in any one of claims 1 and 2, **characterized in that** the clip (5) is made out of a rigid material.

5. Nail as claimed in claim 1, **characterized in that** the added means is/are constituted by at least one bush (2) and at least one clip (5) having arrangements for the engagement of a common attachment screw (4) in the proximal part.

6. Nail as claimed in claim 5, **characterized in that**, where a plurality of bushes (2) is involved, these are formed on the basis of a sleeve (3) capable of being fitted onto the periphery of said nail (1), the branches (5a) and (5b) of the clip being engaged around the sleeve (3), in the gap left free by two bushes, said sleeve (3) having through holes (3a) for the screws (4) in correspondence with holes (1c) in the nail.

7. Nail as claimed in claim 6, **characterized in that** the sleeve (3) is made out of an absorbable material.

8. Nail as claimed in any one of claims 1 to 7, **characterized in that** the distal end (1b) of the nail is in the shape of a square form pointed tip to enable a percutaneous approach.

9. Nail as claimed in claim 1, **characterized in that** the clearance hole or holes (1c) receiving the screw or screws (4) has/have a one pitch thread which acts as a nut-screw assembly.

## Patentansprüche

1. Zentromedullärer Osteosynthesenagel zur Einführung in den Markkanal eines Knochens bei Befestigung im proximalen und eventuell im distalen Teil durch Schrauben (4), wobei der besagte Nagel im Bereich seines proximalen Teils (1a) eine oder mehrere Einrichtungen aufweist, die geeignet sind, einerseits die Zentrierung und seitliche Verkeilung des Nagels (1) in dem besagten Kanal und andererseits die Steifigkeit des humeralen Kopfes gegenüber dem Knochenkörper zu gewährleisten,
**dadurch gekennzeichnet, dass** die angesetzte(n) Einrichtung(en) aus einer Klammer oder mehreren Klammern (5) besteht/bestehen.

2. Nagel nach Anspruch 1, **dadurch gekennzeichnet, dass** die Klammer (5) mindestens zwei Schenkel (5a und 5b) aufweist, die durch einen gemeinsamen Teil (5c) mit Vorkehrungen für die Einführung bzw. Befestigung der Befestigungsschrauben (4) im proximalen Teil verbunden sind.

3. Nagel nach einem der Ansprüche 1 und 2, **dadurch gekennzeichnet, dass** die Klammer (5) aus einem elastischen Werkstoff mit Formgedächtnis hergestellt ist.

4. Nagel nach einem der Ansprüche 1 und 2, **dadurch gekennzeichnet, dass** die Klammer (5) aus einem starren Werkstoff hergestellt ist.

5. Nagel nach Anspruch 1, **dadurch gekennzeichnet, dass** das/die angesetzte(n) Einrichtung(en) aus mindestens einem Ring (2) und mindestens einer Klammer (5) mit Vorkehrungen für das Einführen einer gemeinsamen Befestigungsschraube (4) im proximalen Teil besteht/bestehen.

6. Nagel nach Anspruch 5, **dadurch gekennzeichnet, dass** im Falle mehrerer Ringe (2) letztere aus einer Hülse (3) ausgebildet sind, die geeignet ist, auf den Kreisumfang des besagten Nagels (1) gesteckt zu werden, wobei die Schenkel (5a und 5b) der Klammer um die Hülse (3) herum in den durch zwei Ringe frei gelassenen Zwischenraum eingeführt werden, wobei die besagte Hülse (3) entsprechend zu den Löchern (1c) des Nagels Löcher (3a) für die Durchführung der Schrauben (4) aufweist.

7. Nagel nach Anspruch 6, **dadurch gekennzeichnet, dass** die Hülse (3) aus einem resorbierbaren Material hergestellt ist.

8. Nagel nach einem der Ansprüche 1 - 7, **dadurch gekennzeichnet, dass** das distale Ende (1b) des Nagels mit einer quadratischen Spitze ausgebildet ist, um eine perkutane Annäherung zu ermöglichen.

9. Nagel nach Anspruch 1, **dadurch gekennzeichnet, dass** das bzw. die Durchführungslöcher (1c) für die Aufnahme der Schraube(n) (4) ein Gewinde mit 1 Steigung, das als Mutter-Schraube-System dient, aufweist/aufweisen.
